**Europäisches Patentamt**

**European Patent Office**

**Office européen des brevets**

(11) Veröffentlichungsnummer: **0 238 086**
**A2**

(12) # EUROPÄISCHE PATENTANMELDUNG

(21) Anmeldenummer: **87104052.3**

(22) Anmeldetag: **19.03.87**

(51) Int. Cl.³: **A 61 B 5/02**
**A 61 B 7/04, G 06 F 15/42**

(30) Priorität: **21.03.86 DE 3609567**

(43) Veröffentlichungstag der Anmeldung:
**23.09.87 Patentblatt 87/39**

(84) Benannte Vertragsstaaten:
**AT CH DE FR GB IT LI**

(71) Anmelder: **CONTRAVES GmbH**
**Winterspürerstrasse 17-19**
**D-7768 Stockach(DE)**

(72) Erfinder: **Lang, Erich, Prof. Dr.**

**D-8521 Spardorf(DE)**

(72) Erfinder: **Hubmann, Max, Dr.**
**Rathsberger Strasse 24**
**D-8520 Erlangen(DE)**

(72) Erfinder: **Köppel, Hans**
**Johann-Peter-Hebel-Strasse 8**
**D-6902 Sandhausen(DE)**

(72) Erfinder: **Menke, Franz**
**Am Mühlrain 34**
**D-6903 Neckargemünd(DE)**

(74) Vertreter: **Muschka, Wilhelm, Dipl.-Ing.**
**Baumeister Rohrer Weg 5**
**D-7570 Baden-Baden(DE)**

(54) **Einrichtung zur Bestimmung elastischer Eigenschaften von Arterien.**

(57) Einrichtung zur Bestimmung elastischer Eigenschaften von Arterien unter Ausnutzung ihrer mechanischen Eigenfrequenz, die ein Maß für den Härtewert der Arterie ist. Um Störeinflüsse örtlich benachbarter Organe und/oder Apparaturen auszuschließen und wissenschaftlich eindeutige sowie reproduzierbare Daten zu liefern, bedient man sich eines den systolischen und den diastolisch Druck in den Arterien ermittelnden herkömmlichen Blutdruckmeßgerätes, Aus den ermittelten Blutdruckwerten und der Pulsform der Arterie bestimmt ein programmierbarer Rechner mit Speicher ihre Eigenfrequenz, die Rückschlüsse auf den Härtegrad der Arterien zuläßt.

EP 0 238 086 A2

C O N T R A V E S  GmbH
Winterspürerstr. 17-19
D-7768 Stockach

Beschreibung

## Einrichtung zur Bestimmung elastischer Eigenschaften von Arterien

Die Erfindung betrifft eine Einrichtung zur Bestimmung elastischer Eigenschaften von Arterien nach dem Oberbegriff des Anspruchs 1.

Eine solche Einrichtung ist Gegenstand der DE-OS 27 41 338 und des Fachaufsatzes "Untersuchungen einer neuen Methode zur Beurteilung der arteriellen Gefäßwände" von E. Lang, R. Weinzierl, D. Schilling, M. Hubmann und A. Hullard in "actuelle gerontologie", Seiten 17-19, Januar 1972, Georg Thieme Verlag, Stuttgart 1, Postfach 732. In beiden Fällen wird ein Geber mechanischer Schwingungen in der Nachbarschaft der zu untersuchenden Arterie angelegt und mittels eines mechanischen Abtasters die Amplitude der Schwingungen an der Hautoberfläche nahe dem Aufsetzpunkt des Gebers und damit auch der Arterie gemessen sowie mittels eines 2-Koordinatenschreibers als Funktion der Frequenz des Gebers aufgezeichnet. In dem Fachaufsatz ist drüberhinaus die Forderung nach einer einfachen Handhabung und einer möglichst geringen Belästigung des Patienten erhoben. Ganz allgemein gilt, daß man medizinische Daten möglichst ohne Eingriff in den Körper und seine Funktion erhalten möchte.

*Leerlauf- und Meßkurven dieser Einrichtung besitzen eine vorwiegend wellige Form und sind nicht eindeutig, weil nämlich bei dieser Methode neben den gesuchten Extremwerten noch solche benachbarter Organe - einerseits und/oder möglicherweise auch der hierbei zum Einsatz gelangenden Apparaturen andererseits in die Messungen Eingang finden.*

*Der Beitrag "6. Biomechanik des Blutkreislaufes" von Martin Rödenbeck im "Biophysikalische Grundlagen der Medizin", Gustav Fischer Verlag Stuttgart, New York, 1980, Seiten 244-289, geht, vor allem auf Seite 246 untere Hälfte, auf die Elastizität der Gefäßwände und die Bedeutung ihrer altersbedingten Veränderung hinsichtlich der Funktionstüchtigkeit näher ein.*

*Die Aufgabe der Erfindung wird in einer Verbesserung der gattungsgemäßen Einrichtung dahingehend gesehen, Störeinflüsse auszuschalten sowie wissenschaftlich eindeutige und reproduzierbare Daten zu liefern. Gemäß der Erfindung wird diese Aufgabe durch die im Kennzeichen des Anspruchs 1 genannten Merkmale gelöst. Hierbei macht man sich die vom Innendruck der Arterie abhängige Dehnbarkeit zunutze, deren Eigenfrequenz bei zunehmender Dehnung gleichfalls zunimmt. Auch das Blutvolumen im Meßabschnitt erhöht sich und wirkt der Eigenfrequenz entgegen. Der steigende Elastizitätsmodul der Arterie wird aber nicht kompensiert, so daß sich bei Systole und Diastole unterschiedliche Eigenfrequenzen ergeben. Eine entsprechende Einrichtung gewährleistet die schnelle Auswertung der hierzu erforderlichen Meßkurven.*

*Die Unteransprüche sehen vorteilhafte Weiterbildungen der Erfindung vor.*

*Im folgenden wird die Erfindung an Hand eines Ausführungsbeispieles näher erläutert.*

*Mit 1 ist ein Blutdruckmeßgerät herkömmlicher Art bezeichnet, dessen um den Arm eines Patienten gelegte Manschette 2 über den Ballon 3 aufgepumpt, mittels der Sperrschraube 4 das erreichte Druckniveau zunächst festgehalten sowie anschließend dosiert wieder abgelassen wird, wobei auf dem Gerät 5 der Blutdruck angezeigt und abgelesen werden kann.*

*Das Blutdruckmeßgerät 1 ist über eine Anpassungselektronik 6 einmal mit dem programmierbaren sowie mit einem Speicher versehenen Rechner 7 und zum anderen mit dem Schirmbildsichtgerät 8 funktionell verbunden. Für die Blutdruckanzeige enthält es ein Mikrophon, das gleichzeitig für die Darstellung der Pulskurve verwendet wird. Diese Funktion kann bei einem anderen Ausführungsbeispiel auch von einem zusätzlichen Mikrophon übernommen werden, das entweder über das Gerät 5 oder direkt über die Anpassungselektronik 6 Signale an den Rechner 7 liefert. Das zweite Mikrophon ist sinnvoll, da mit ihm eine vorspannungsfreie Messung möglich wird.*

*Mit dem Rechner steht auch noch der Schreiber oder Drucker 9 in Verbindung. Auswerteelektronik, Rechner, Schirmbildsichtgerät und Drucker lassen sich bei einem anderen, zeichnerisch nicht dargestellten Ausführungsbeispiel in einer einzigen Baueinheit zusammenfassen.*

*Was die Funktion dieser Einrichtung anbetrifft, so ermittelt der Rechner 7 aus der Pulsform der Gefäße sowie aus den Blutdruckwerten bei systolischem und diastolischem Druck die Eigenfrequenz der Arterie. Die Werte werden aus der ansteigenden Flanke der Amplitude des Pulses gewonnen, und zwar aus dem oberen steilen Teil einer Amplitude die Eigenfrequenz des systolischen Drucks und anschließend aus dem flacheren unteren Teil der Amplitude die Eigenfrequenz des diastolischen Drucks. Ferner ermittelt der Rechner aus diesen beiden Frequenzen die arterielle Resonanzfrequenz und ihr Verhältnis zum Lebensalter. Die Eigenfrequenzen erlauben Rückschlüsse auf den Härtegrad der Arterien. Sämtliche errechneten Werte können auf dem Schirmsichtgerät 8 angezeigt und über den Drucker 9 auch schriftlich fixiert werden.*

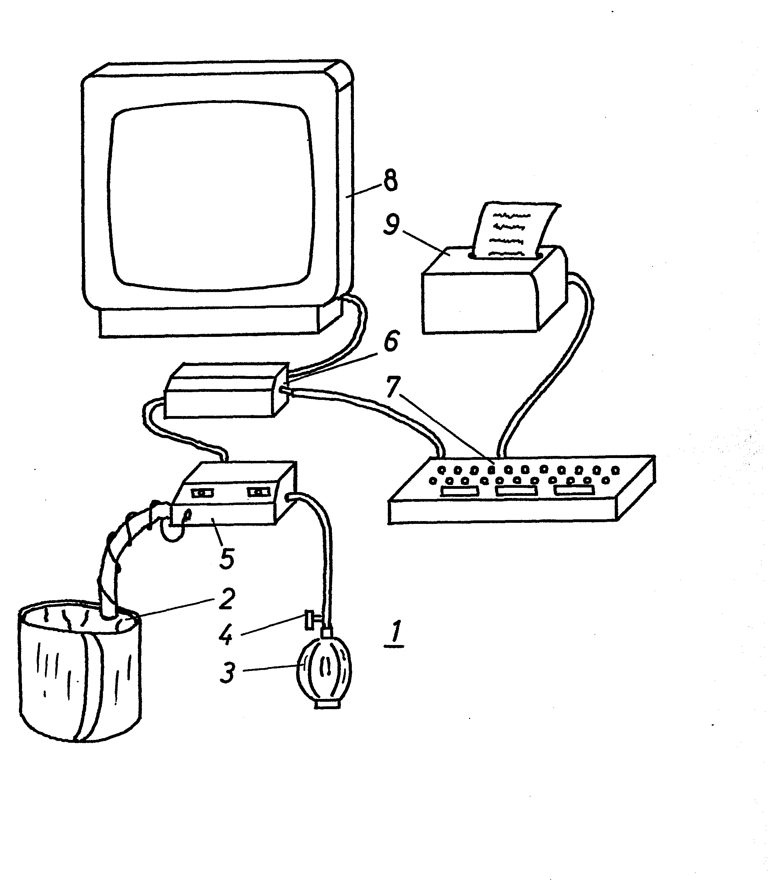

Patentansprüche

1. Einrichtung zur Bestimmung elastischer Eigenschaften von Arterien unter Ausnutzung ihrer mechanischen Eigenfrequenz, die mit einem Schreiber zur Aufzeichnung, verbunden ist, d a d u r c h g e k e n n z e i c h n e t, daß

a) ein den systolischen und den diastolischen Druck in den Arterien ermittelndes herkömmliches Blutdruckmeßgerät (1) mit integriertem Mikrophon Verwendung findet und

b) das Blutdruckmeßgerät (1) mit einem aus den Blutdruckwerten und der Pulsform der Gefäße ihre Eigenfrequenz bestimmenden programmierbaren Rechner mit Speicher (7) verbunden ist.

2. Einrichtung nach Anspruch 1, d a d u r c h g e k e n n z e i c h n e t, daß der mit einem Speicher versehene Rechner (7) zum Zweck der Anzeige der ermittelten Werte sowohl mit einem Drucker (9) als auch mit einem Schirmbildsichtgerät (8) verbunden ist.

3. Einrichtung nach Anspruch 1 und 2, d a d u r c h g e k e n n z e i c h - n e t, daß zwischen Blutdruckmeßgerät (1) und Rechner (7) einerseits sowie zwischen Blutdruckmeßgerät (1) und Schirmbildsichtgerät (8) verzugsweise eine gemeinsame Anpassungselektronik (6) geschaltet ist.

4. Einrichtung nach einem der vorausgehenden Ansprüche, d a d u r c h g e k e n n z e i c h n e t, daß für die Darstellung der Pulskurve das erste oder ein weiteres Mikrophon verwendet wird.

5. Einrichtung nach Anspruch 4, d a d u r c h g e k e n n z e i c h n e t, daß das zweite Mikrophon sein Signal über das Gerät (5) oder direkt über die Anpassungselektronik (6) an den Rechner (7) liefert.

6. Einrichtung nach einem der vorausgehenden Ansprüche, d a d u r c h g e - k e n n z e i c h n e t, daß Anpassungselektronik (6), Rechner (7), Schirmbildsichtgerät (8) und Drucker (9) in einer Baueinheit zusammengefaßt sind.